# EUROPEAN PATENT SPECIFICATION

(11) **EP 1 426 027 B1**
(45) Date of publication and mention of the grant of the patent: **05.09.2012**
(21) Application number: 01965641.2
(22) Date of filing: 14.09.2001
(51) Int. Cl.: A61K 8/891, A61K 8/92, A61Q 19/00, A61Q 5/12, A61K 8/31, A61K 8/37, A61K 8/91, A61K 8/895

(54) **COMPOSITION AND COSMETIC PREPARATION CONTAINING THE SAME**
ZUSAMMENSETZUNG UND DIESE ENTHALTENDE KOSMETISCHE ZUBEREITUNG
COMPOSITION ET PREPARATION COSMETIQUE CONTENANT LADITE COMPOSITION

(43) Date of publication of application: 09.06.2004
(73) Proprietor: Shin-Etsu Chemical Company, Ltd., Tokyo 100-0004 (JP); Sakuta, Koji, Usuigun, Gunma 379-0224 (JP)
(72) Inventor: SAKUTA, K., Silicone Electronic Material Res. Ctr, Usuigun, Gunma 379-0224 (JP)
(74) Representative: Solf, Alexander
(86) International application number: PCT/JP2001/007987
(87) International publication number: WO 2003/024413

(56) References cited:
- EP-A- 0 850 640
- EP-A- 0 908 175
- EP-A2- 0 295 886
- EP-A2- 0 381 166
- EP-A2- 1 074 576
- JP-A- 61 194 009
- JP-A- 2001 213 966
- US-A- 4 987 169
- US-A- 5 266 321

## Description

### Field of the Invention

This invention relates to a composition containing a crosslinking type organopolysiloxane and an oil other than a silicone oil, and to a cosmetic preparation containing this composition. More specifically, it relates to a cosmetic preparation, which due to containing the aforesaid composition, spreads lightly and cleanly, has plasticity, flatness, and smoothness, and imparts an emollient effect when it is applied. It excels in a wide spectrum of effect from natural gloss to a matte feeling, and has good stability over time.

### Background of the Invention

In the prior art, cosmetic preparations with the addition of liquid oils such as paraffins, esters, glycerides, animal oils, vegetable oils and higher alcohols were used to impart plasticity, flatness and smoothness, and to give an emollient effect, but these cosmetic preparations had the fault that oiliness, tackiness and the feeling of an oil film could not be avoided. A cosmetic preparation which blended a silicone oil such as dimethylpolysiloxane with the aforesaid cosmetic preparation to suppress oiliness, tackiness and the feeling of an oil film, is known.

Silicone oils have good spreading qualities, and outstanding flatness, smoothness and water repellence, but there is a limit to their compatibility with oils other than silicone oils, and in order to ensure the stability of the cosmetic preparation with which they are blended, the silicone oils must be solidified or thickened, and the other oils must also be solidified or thickened. Various moieties have been proposed to solidify or thicken silicone oils, e.g., a compound in which alkyl groups are introduced into the silicone chain (Tokkai Hei 02-64115), a compound in which an aliphatic alcohol having 21-30 carbon atoms or an ester group of an acid is introduced into the silicone chain (Tokkai Hei 10-500431), an acrylate silicone (Tokkai Hei 02-132141), or a crosslinking type organopolysiloxane (Tokkyo 2105655), and various trials are being carried out.

Examples of substances used to solidify or thicken liquid oils other than silicone oils such as paraffins, esters, glycerides, animal oils, vegetable oils and higher alcohols, are solid waxes having a high melting point, metallic soaps, starch fatty acid esters or organic-modified clay minerals.

However, the solubility of metallic soaps in liquid oils is poor, and as a high temperature of about 100°C is required for dissolution, the quality of other mixed oils and pigments deteriorates, while cracks and oil bleeding develop over time and sweating tends to occur during use. Although starch fatty acid esters are excellent as gelating agents, they suffer from the disadvantage that oil separates over time. In any case, these solidifying agents are oily or tacky, feel like an oil film and tend to become lumpy, so liquid is released over time and it is difficult to keep them stable from low temperature to high temperature, hence they were not completely satisfactory.

Compositions for cosmetics comprising partially crosslinked organopolysiloxane and liquid oil disclosed in EP 0980175 and EP 0850640 are not completely satisfactory in stability.

In addition, crosslinked organopolysiloxane particles having good affinity for organic oil were disclosed in EP 1074576. However, the particles were used for not preparing a pasty composition but preparing an emulsion.

The Inventor, as a result of intensive studies aimed at resolving the, aforesaid disadvantages, discovered that a composition as defined in claim 1 comprising a crosslinking type organopolysiloxane and an oil other than a silicone oil was suitable for cosmetic preparations. In particular, it was discovered that the aforesaid crosslinking type organopolysiloxane should be a specific crosslinking type organopolysiloxane as defined in claim 1 which swells up with at least its own weight of an oil other than a silicone oil. By blending this composition with a cosmetic preparation, a cosmetic preparation which is pleasant to use, easy to use and very stable over time is obtained, and this led to the present invention.

It is therefore a first object of this invention to provide a composition suitable for a cosmetic preparation which spreads easily and has a fresh feeling, and which in particular is not tacky or heavy when applied, spreads smoothly and lightly, and leaves the skin feeling clean and smooth.

It is a second object of the invention to provide a cosmetic preparation which excels in usability, i.e., which does not prevent suitable transpiration when applied, which imparts flexibility, smoothness and emollient qualities, and which can give a wide range of finishes from natural gloss to matt, as well as having good stability over time.

### Summary of the Invention

The aforesaid objects of the invention are attained by a composition as defined in claim 1 comprising a crosslinking type organopolysiloxane and an oil other than a silicone oil, and by a cosmetic preparation containing this composition.

The cosmetic preparation which contains a blend of a composition comprising the crosslinking type organopolysiloxane polymer of this invention and an oil other than a silicone oil spreads easily and has a fresh feeling, and in particular is not tacky or heavy when applied, spreads lightly, and leaves the skin feeling clean and smooth. Therefore, the cosmetic preparation of this invention has excellent usability, i.e., when the cosmetic preparation of this invention is applied, it imparts flexibility, smoothness and emollient qualities without preventing a suitable degree of transpiration and it can give a wide range of finishes from natural gloss to matt, as well as having good stability over time.

### Description of the Preferred Embodiments

The crosslinking type organopolysiloxane of the composition of this invention is a crosslinking type organopolysiloxane which swells up with at least its own weight of an oil other than a silicone oil.

In this invention, the crosslinking type organopolysiloxane is a crosslinking type organopolysiloxane obtained by the addition polymerization of an organohydrogen polysiloxane expressed by the following formula (1) with an organopolysiloxane expressed by the following formula (2)
(1) : organohydrogenpolysiloxane comprising at least one type of structural unit selected from groups comprising a SiO₂ unit, HSiO_{1.5} unit, RSiO_{1.5} unit, RHSiO unit, R₂SiO unit, R₃SiO_{0.5} unit and R₂HSiO_{0.5} unit (herein, R is a monofunctional hydrocarbon group having 1-30 carbon atoms which may be substituted or unsubstituted, but is not an aliphatic unsaturated group,), and comprising on average 1.5 or more hydrogen atoms bonded to a silicon atom in the molecule.
(2) : organopolysiloxane comprising at least one type of structural unit selected from among a SiO₂ unit, (CH₂=CH)SiO_{1.5} unit, RSiO_{1.5} unit, R(CH₂=CH)SiO unit, R₂SiO unit, R₃SiO_{0.5} unit and R₂(CH₂=CH)SiO_{0.5} unit (wherein, R is identical to that of the aforesaid (1)), and comprising on average 1.5 or more vinyl groups bonded to a silicon atom in the molecule.

Herein, the organopolysiloxane expressed by (1) or (2) is selected so that the total content of at least one of the hydrogen atoms bonded to silicon and the vinyl groups bonded to silicon is less than 20 mol% of the sum of all organic groups and hydrogen atoms bonded to silicon.

The organohydrogenpolysiloxane of (1) may comprise a SiO₂ unit, HSiO_{1.5} unit, RSiO_{1.5} unit, RHSiO unit, R₂SiO unit, R₃SiO_{0.5} unit and R₂HSiO_{0.5} unit. R is a monofunctional hydrocarbon group having 1-30 carbon atoms which may be substituted or unsubstituted, but is not an aliphatic unsaturated group. Examples of R are alkyl groups such as methyl, ethyl, propyl, lauryl, myristyl, palmityl and stearyl; aryl groups such as phenyl and tolyl; monofunctional hydrocarbons comprising cycloalkyl groups such as cyclohexyl groups, and substituted hydrocarbon groups wherein one, two or more hydrogen atoms in these monofunctional hydrocarbon groups are substituted by a halogen atom such as chlorine, bromine or fluorine, or a cyano group, e.g., a γ-trifluoropropyl group or chloromethyl group. In particular, it is preferred that 30 mol% or more of R is a methyl group and 5-50 mol% is a hydrocarbon group having 10-22 carbon atoms, but more preferred that the hydrocarbon group having 10-22 carbon atoms accounts for 10-40 mol%. Further, this organohydrogenpolysiloxane expressed by (1) may be straight-chain, branched or cyclic.

The organohydrogenpolysiloxane expressed by (1) preferably contains on average 1.5 or more, more preferably 2-50 and most preferably 2-10 hydrogen atoms bonded to a silicon atom (Si-H) in the molecule. The proportion of hydrogen atoms bonded to silicon atoms in the molecule is normally 0.5-50 mol%, but more preferably 1-20 mol% relative to the sum of hydrogen atoms and all organic groups bonded to a silicon atom.

The vinyl group-containing organopolysiloxane expressed by (2) comprises at least one type of structural unit selected from a group comprising a SiO₂ unit, (CH₂=CH)SiO_{1.5} unit, RSiO_{1.5} unit, R(CH₂=CH)SiO unit, R₂SiO unit, R₃SiO_{0.5} unit and R₂(CH₂=CH)SiO_{0.5} unit (wherein, R is identical to that of the aforesaid (1)). Further, the molecule preferably contains on average 1.5 or more, but more preferably 2-50 vinyl groups bonded to a silicon atom. The average number of vinyl groups bonded to a silicon atom in the molecule is preferably 2-10, but more preferably 2-5. It is preferred that 30 mol% or more of R is a methyl group and 5-50 mol% is a hydrocarbon group having 10-22 carbon atoms, but more preferred that the hydrocarbon group having 10-22 carbon atoms accounts for 10-40 mol%.

The molecular structure of the organopolysiloxane containing the vinyl group expressed by (2) may be straight-chain, branched or cyclic. The amount of this vinyl group in the molecule is preferably 0.5-50 mol%, but more preferably 1-20 mol% of all organic groups bonded to a silicon atom.

As mentioned above, at least one of the organohydrogen polysiloxane expressed by (1) and the vinyl group-containing organopolysiloxane expressed by (2) is selected so that the reactive groups (hydrogen atoms and vinyl groups bonded to a silicon atom) in the molecule account for less than 20 mol% relative to the sum of all organic groups and hydrogen atoms bonded to a silicon atom. As a result, the degree of crosslinking of the polymer obtained by the addition polymerization is satisfactory, and a suitable swelling quality relative to the oil is obtained.

R in the crosslinking type organopolysiloxane comprising the organohydrogenpolysiloxane expressed by (1) and the vinyl group-containing organopolysiloxane expressed by (2) is a monofunctional hydrocarbon group having 1-30 carbon atoms which may be substituted or unsubstituted, but is not aliphatic unsaturated groups, and part of R is preferably a monofunctional hydrocarbon group having 10-22 carbon atoms which may be substituted or unsubstituted except by aliphatic unsaturated groups. In this way, a composition comprising the crosslinking type organopolysiloxane polymer and oil other than a silicone oil, can be stably obtained.

The addition polymerization of the organohydrogenpolysiloxane expressed by (1) with the organopolysiloxane expressed by (2) may be performed in the absence of a solvent, but an organic solvent may also be used if required. Examples of this organic solvent are aliphatic alcohols such as methanol, ethanol, 2-propanol and butanol, aromatic hydrocarbons such as benzene, toluene and xylene, aliphatic or cyclic hydrocarbons such as n-pentane, n-hexane and cyclohexane, and halogenated hydrocarbons such as dichloromethane, chloroform, carbon tetrachloride, trichloroethane and fluorinated hydrocarbons.

Examples of the catalyst for the aforesaid addition polymerization areplatinumcompounds (e.g.,chloroplatinic acid, alcohol-modified chloroplatinic acid or chloroplatinic acid-vinyl siloxane complex), or rhodium compounds. The addition polymerization may be performed by reaction in the presence of this catalyst at room temperature or with heating (about 50-150°C). Examples of catalysts which are particularly preferred in this invention are chloroplatinic acid and the platinum compounds such as [Pt(PPh₃)₃] used for hydrosilylation reactions mentioned in US Patents Nos. 3,159,601; 3,159, 662; and 3,775,452. The aforesaid platinum compound may for example be a complex of a vinylsiloxane with a platinum compound, or such a compound which has been modified by an alcohol. In this invention, the use of the chloroplatinic acid mentioned in Tokkosho 33-9969 or a complex of a vinylsiloxane and chloroplatinic acid is particularly preferred.

This addition polymerization may for example be performed by blending predetermined amounts of the component expressed by (1) with the component expressed by (2) in a reactor such as a planetary mixer equipped with a stirrer, adding the catalyst, and stirring at a suitable temperature of about 50-150°C. In this way, the desired crosslinking type organopolysiloxane can easily be obtained.

The oil other than a silicone oil in the composition of this invention may be suitably selected from among liquid oils known in the art which are usually used in cosmetic preparations.

A hydrocarbon oil, for example, may be a straight-chain or branched hydrocarbon oil, or a volatile hydrocarbon oil. Examples of this hydrocarbon oil are an α-olefin oligomer, light isoparaffin, light liquid isoparaffin, squalane, synthetic squalane, vegetable squalane, squalen, liquid paraffin and liquid isoparaffin. Examples of an ester oil which can be mixed therein include diisobutyl adipate, 2-hexyldecyl adipate, di-2-heptylundecyl adipate, N-alkylglycol monoisostearates, isocetyl isostearate, trimethylolpropane triisostearic acid ester, ethylene glycol di-2-ethylhexanoic acid ester, cetyl 2-ethylhexanoate, trimethylolpropane tri-2-ethylhexanoic acid ester, pentaerythritol tetra-2-ethylhexanoic acid ester, cetyl octanoate, octyldodecyl gum ester, oleyl oleate, octyldodecyl oleate, decyl oleate, neopentyl glycol dicapric acid ester, triethyl citrate, 2-ethylhexyl cinnamate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, N-lauroyl-L-glutaminic acid 2-octyldodecyl ester and diisostearyl malic acid.

Examples of glyceride oils are acetoglyceryl, glyceryl trioctanoate, glyceryl triisostearate, glyceryltriisopalmitate, glyceryl monostearate, glyceryl di-2-heptyl undecanoate, glyceryl trimyristate and diglyceryl myristate-isostearate. Examples of higher fatty acids are undecylenic acid, oleic acid, linolic acid, linolenic acid, arachidonic acid, eicosapentaenoic acid (EPA), docosa-hexaenoic acid (DHA), isostearic acid and lactic acid, and examples of higher alcohols are oleyl alcohol, isostearyl alcohol, hexyl decanol, octyl dodecanol, cetostearyl alcohol, 2-decyltetradecinol and monooleyl glyceryl ether (selachyl alcohol).

Examples of natural animal and vegetable oils and fats, and semi-synthetic oils and fats, are avocado oil, almond oil, olive oil, liver oil, beef foot tallow, apricot kernel oil, wheat germ oil, sesame oil, rice germ oil, rice bran oil, sasanqua oil, safflower oil, cinnamon oil, turtle oil, soybean oil, tea fruit oil, camellia oil, evening primrose oil, corn oil, rapeseed oil, Japanese kiri oil, germ oil, par chic oil, castor oil, castor oil fatty acid methyl ester, sunflower seed oil, grape oil, jojoba oil, macadamia nut oil, mink oil, medoform oil, cotton seed oil, tricoconut oil fatty acid glycerides, arachis oil, liquefied lanolin, acetic acid lanolin alcohol, lanolin fatty acid polyethylene glycol and egg yolk oil.

Of these liquid oils, in this invention, the use of a hydrocarbon oil, ester oil, glyceride oil, natural animal or vegetable oil and semi-synthetic oil is preferred, and of the hydrocarbon oils, the use of a branched hydrocarbon oil or volatile hydrocarbon oil is preferred.

The composition of this invention can easily be obtained by adding the oil other than a silicone oil to the crosslinking type organopolysiloxane obtained by the aforesaid method with stirring. The composition may be in the form of any of a powder, paste or liquid. The composition of this invention can also be manufactured directly by having the oil other than a silicone oil co-present together with the organic solvent when the addition polymerization reaction is performed to manufacture the aforesaid crosslinking type organopolysiloxane. The oil can also be added later to this composition. In any case, the crosslinking type organopolysiloxane which is one component of the composition of this invention is insoluble in the oil other than a silicone oil, but is a crosslinking type organopolysiloxane with the particular quality that it swells up by containing at least its own weight of said oil.

The blending proportion of the crosslinking type organopolysiloxane and the oil other than a silicone oil in the composition of this invention is preferably 1/20-20/1, but more preferably 1/10-1/1 (weight ratio).

The composition of this invention may be used for various purposes, but is particularly suitable as a composition of all cosmetic preparations used for the skin and hair. Examples of cosmetic preparations used for the skin and hair are skin care cosmetic preparations such as a milky lotion, cream, cleansing, pack, oil liquid, massage material, beautifying lotion, washing agent, deodorant, hand cream and lip cream, makeup cosmetic preparations such as makeup foundation, white powder, liquid foundation, oil-based foundation, rouge, eye shadow, mascara, eyeliner, eyebrow and lipstick, hair cosmetic preparations such as shampoo, rinse, treatment and setting agent, antiperspirant, and ultraviolet cut cosmetic preparations such as sunscreen lotion and sunscreen cream. In this case, the blending amount of the composition of this invention which is component A) differs depending on the type of cosmetic preparation, but can be within the range of 0.1-95wt% and more preferably within the range of 2-80wt% of the total amount of cosmetic preparation.

The cosmetic preparation of this invention may also contain one, two or more oils other than the liquid oil contained in the composition which is component A), blended in as component B). The oil which is component B) may be a solid, semisolid or liquid oil, provided that it is used ordinary cosmetic preparations, Examples of natural animal and vegetable oils and fats, and semi-synthetic oils and fats, are avocado oil, linseed oil, almond oil, Chinese wax, perilla oil, olive oil, cacao butter, kapok wax, kaya oil, carnauba wax, liver oil, candellila wax, beef tallow, beef foot oil, beef bone fat, hydrogenated beef tallow, apricot kernel oil, spermaceti, hydrogenated oil, wheat germ oil, sesame oil, rice germ oil, rice bran oil, sugar cane wax, sasanqua oil, safflower oil, shea butter, Chinese tung oil, cinnamon oil, jojoba wax, shellac wax, turtle oil, soybean oil, tea seed oil, tsubaki oil, evening primrose oil, corn oil, lard, rape seed oil, Japanese tung oil, rice-bran wax, germ oil, horse fat, persic oil, palm oil, palm kernel oil, castor oil, hydrogenated castor oil, methyl caster oil fatty acid, sunflower oil, grape seed oil, bayberry wax, jojoba oil, macadamia nut oil, beeswax, mink oil, cottonseed oil, cotton wax, Japan wax, haze kernel oil, montan wax, coconut oil, hydrogenated coconut oil, tricoconut oil fatty acid glyceride, mutton-tallow, peanut oil, lanolin, liquid lanolin, reduced lanolin, lanolin alcohol, hard lanolin, lanolin acetate, lanolin fatty acid isopropyl, hexyl laurate, POE lanolin alcohol ether, POE lanolin alcohol acetate, polyethylene glycol lanolin fatty acid, POE hydrogenated lanolin alcohol ether, and egg yolk oil. POE means polyoxyethylene.

Examples of hydrocarbon oils include ozokerite, α-olefin oligomer, light isoparaffin, light liquid paraffin, squalane, synthetic squalene, vegetable squalane, squalane, sericine, paraffin, paraffin wax, liquid paraffin, liquid isoparaffin, pristane, polyisobutylene, microcrystalline wax and vaseline; and those of a higher fatty acid which can be mixed include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid and 12-hydroxystearic acid. Examples of a higher alcohol include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, POE cholesterol ether, monostearyl glycerin ether (batyl alcohol) and monooleyl glyceryl ether (cerakyl alcohol).

Examples of an ester oil include diisobutyl adipate, 2-hexyldecyl adipate, di-2-heptylundecyl adipate, N-alkylglycol monoisostearates, isocetyl isostearate, trimethylolpropane triisostearic acid ester, ethylene glycol di-2-ethylhexanoic acid ester, cetyl 2-ethylhexanoate, trimethylolpropane tri-2-ethylhexanoic acid ester, pentaerythritol tetra-2-ethylhexanoic acid ester, cetyl octanoate, octyldodecyl gum ester, oleyl oleate, octyldodecyl oleate, decyl oleate, neopentyl glycol dicapric acid ester, triethyl citrate, 2-ethylhexyl cinnamate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, N-lauroyl-L-glutaminic acid 2-octyldodecyl ester and diisostearyl malic acid; and examples of glyceride oil which can be mixed therein include acetoglyceride, triisooctanoic acid glyceride, triisostearic acid glyceride, triisopalmitic acid glyceride, tri-2-ethylhexanoic acid glyceride, monostearic acid glyceride, di-2-heptylundecanoic acid glyceride, trimyristic acid glyceride and myristic acid isostearic acid diglyceride.

As examples of silicone oils which can be mixed therein, mention may be made of organopolysiloxanes having from low to high viscosities, such as dimethylpolysiloxane, methylphenylpolysiloxane, methylhydrogenpolysiloxane and dimethylsiloxane-methylphenylsiloxane copolymer; cyclic siloxanes, such as octamethylcyclotetrasiloxane, decamethylcyclopentasiloxane, dodecamethylcyclohexasiloxane, tetramethyltetrahydrogencyclotetrasiloxane; silicone rubbers, such as gummy dimethylpolysiloxanes having high polymerization degrees and gummy dimethylsiloxane-methylphenylsiloxane copolymers having high polymerization degrees; and cyclosiloxane solutions of silicone rubber, trimethylsiloxysilicate, cyclosiloxane solutions of trimethylsiloxysilicate, higher alkoxy-modified silicones such as stearoxysilicone, higher fatty acid-modified silicones, alkyl-modified silicones, amino-modified silicones, fluorine-modified silicones, silicone resins and silicone resin solutions. Examples of fluorinated oils are fluoropolyether, perfluorodecalin and perfluorooctane. The blending amount of these oils, which is component B), may be 1-98wt% of the whole cosmetic preparation.

Water can be blended with the cosmetic preparation of this invention as component C) according to the purpose. The blending amount may be 1-95wt% of the whole cosmetic preparation. One, two or more of compounds having an alcoholic hydroxyl group in the molecular structure can be blended with the cosmetic preparation of this invention as component D) according to the purpose. Examples of compounds having an alcoholic hydroxyl group which can be added in this invention are lower alcohols such as ethanol and isopropanol; sugar alcohols such as sorbitol and maltose; sterols such as cholesterol, sitosterol, phytosterol and lanosterol; and polyhydric alcohols such as butylene glycol, propylene glycol and dibutylene glycol. The blending amount of component D) may be within the range of 0.1-98wt% of the whole cosmetic preparation.

The cosmetic preparation of this invention may also contain one, two or more water-soluble or water-swelling polymers as component E) according to the purpose. Examples are gum arabic, tragacanth, galactan, carob gum, guar gum, karaya gum, carrageenan, pectin, agar, quince seed, starch (rice, corn, potato, wheat), alge colloid, tranto gum and locust bean gum; microbial polymers, such as xanthan gum, dextran, succinoglucan and pullulan; animal polymers, such as collagen, casein, albumin and gelatin; starch polymers, such as carboxymethyl starch and methylhydroxypropyl starch; cellulose polymers, such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethylcellulose, crystalline cellulose and powdery cellulose; alginic acid polymers, such as sodium alginate and propylene glycol ester of alginic acid; vinyl polymers, such as polyvinyl methyl ether and carboxyvinyl polymer; polyoxyethylene polymers; polyoxyethylene-polyoxypropylene copolymers; acrylic polymers, such as sodium polyacrylate, polyethylacrylate and polyacrylamide; other synthetic water-soluble polymers, such as polyethyleneimines and cationic polymers; and inorganic water-soluble polymers, such as bentonite, aluminum magnesium silicate, montmorillonite, beidellite, nontronite, saponite, hectorite and silicic acid anhydride.

In these water-soluble polymers, film-forming agents, such as polyvinyl alcohol and polyvinyl pyrrolidine, are also included. The blending amount of component E) is within the range of 0.1-25wt% of total cosmetic preparation.

One, two or more powders and/or colorants may also be used as component F) in the cosmetic preparation of this invention according to the purpose.

This powder, provided that it is a powder used for ordinary cosmetic preparations, may have any form (spheroidal, acicular, tabular), particle diameter (fume, fine particle, pigment) or particle structure (porous, non-porous). Examples are inorganic powders, organic powders, surfactant metal salt powders, colored pigments, pearl pigments, metal powder pigments and natural pigments.

Examples of inorganic powders are titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, syntheticmica, phlogopite, red mica, biotite, lithia mica, silicic acid, silicic acid anhydride, aluminium silicate, magnesium silicate, magnesium aluminium silicate, calcium silicate, barium silicate, strontium silicate, metal tungstates, hydroxyapatite, vermiculite, haidilite, bentonite, montmorillonite, hectorite, zeolite, ceramic powder, dibasic calcium phosphate, alumina, aluminium hydroxide, boron nitride and silica.

Examples of organic powders are polyamide powder, polyester powder, polyethylene powder, polypropylene powder, polystyrene powder, polyurethane, benzoguanamine powder, polymethyl benzoguanamine powder, tetrafluoroethylene powder, polymethylmethacrylate powder, cellulose, silk powder, nylon powder, nylon 12, nylon 6, crosslinked silicone fine powder having dimethylsilicone crosslinks, polymethylsilsesquioxane fine powder, styrene-acrylic acid copolymer, divinylbenzene-styrene copolymer, vinyl resin, urea resin, phenol resin, fluororesin, silicone resin, acrylate resin, melamine resin, epoxy resin, polycarbonate resin, microcrystalline fiber powder, starch powder and lauroyl lysine.

Examples of surfactant metal salt powders (metallic soaps) are zinc stearate, aluminum stearate, calcium stearate, magnesium stearate, zinc myristate, magnesium myristate, zinc cetyl phosphate, calcium cetyl phosphate and zinc sodium cetyl phosphate. Examples of colored pigments are inorganic red pigments such as iron oxide, iron hydroxide and iron titanate, inorganic brown pigments such as γ-iron oxide, inorganic yellow pigments such as yellow iron oxide and ocher, inorganic black pigments such as black iron oxide and carbon black, inorganic purple pigments such as manganese violet and cobalt violet, inorganic green pigments such as chromium hydroxide, chromium oxide, cobalt oxide and cobalt titanate, inorganic blue pigments such as Berlin blue and ultramarine blue, tar pigment lake, natural pigment lake and synthetic resin powders which are complexes of these powders. Examples of pearl pigments are titanium oxide-coated mica and titanium oxide-coated mica, bismuth oxychloride, titanium oxide-coated bismuth oxychloride, titanium oxide-coated talc, scales foil and titanium oxide-coated colored mica; examples of metal powder pigments are aluminium powder, kappa powder and stainless steel powder; examples of tar dyes are Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206 and Orange No. 207; examples of natural pigments are powders selected from among carminic acid, laccainic acid, carthamin, bradilin and crocin.

Of these powders and/or colorants, at least part is a crosslinking type silicone fine powder having a structured crosslinked by dimethylsilicone, a polymethylsilsesquioxane fine powder, hydrophobic silica or a composite fine powder wherein a spherical silicone rubber surface is coated by polymethylsilsesquioxane particles; and for at least part of the powders and/or colorants, a substance which is a powder and/or colorant having fluorine groups is often used. Also, to the extent that it does not interfere with the effect of the invention, these powders may be complexed or treated with common oils, silicone oils, fluorine compounds or surfactants, one, two or more being be used as required.

The blending amount of the component F) may be within the range of 0.1-99wt% of the total amount of cosmetic preparation. In particular, the blending amount in the case of a powdered solid cosmetic preparation may be in the range of 80-99wt% of the total amount of cosmetic preparation.

In the cosmetic preparation of this invention, one, two or more surfactants may be used as the component G) if required. Examples of this surfactant are anionic, cationic, non-ionic and amphoteric surfactants, but there is no particular limitation in this invention, and they may be suitably selected from among those normally used in cosmetic preparations.

Examples of a usable anionic surfactant include fatty acid soaps, such as sodium stearate or triethanolamine palmitate; alkyl ether carboxylic acids and salts thereof; salts of amino acid-fatty acid condensates; alkanesulfonates; alkenesulfonates; sulfonated fatty acid esters; sulfonated fatty acid amides; sulfonates of formaldehyde condensate type; alkylsulfates; higher secondary alcohol sulfates; alkyl and aryl ether sulfates; fatty acid ether sulfates, fatty acid alkylolamide sulfates; ether sulfates, such as Turkey red oil; alkyl phosphates; ether phosphates; alkyl aryl ether phosphates; amide phosphates; and active agents of N-acylamino acid type.

Examples of a usable cationic surfactant include amine salts, such as alkylamine salts, polyamines and aminoalcohol fatty acid derivatives, quaternary alkylammonium salts, quaternary arylammonium salts, pyridinium salts and imidazolium salts. Examples of a usable nonionic surfactant include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ehter, polyoxyethylene phytosterol ether, polyoxyethylene cholestanol ether, polyoxyethylene cholesteryl ether, polyoxyalkylene-modified organopoly-siloxanes, organopolysiloxanes modified with both polyoxyalkylene and alkyl groups, alkanolamides, sugar ethers and sugar amides; and examples of a usable amphoteric surfactant include betaine, aminocarboxylate, imidazoline derivatives and amidoamines.

Of these surfactants, in this invention, a surfactant which is a straight-chain or branched silicone having a polyoxyalkylene chain in the molecule, and a surfactant having a HLB of 2-8, are often used.

The blending amount of the component G) is preferably 0.1-20wt%, but more preferably 0.2-10wt% of the total amount of cosmetic preparation.

One, two or more crosslinking type organopolysiloxanes which swell up with at least their own weight of a silicone having a low viscosity of 0.65mm²/s (25°C)-100.0mm²/s (25°C) may be used as component H) in the cosmetic preparation of this invention according to the purpose. This crosslinking type organopolysiloxane may be a crosslinking type organopolysiloxane containing at least one part selected from a group comprising a polyoxyalkylene part, alkyl part, alkenyl part, aryl part and fluoroalkyl part. The blending amount when this crosslinking type organopolysiloxane which is component H) is used, is preferably 0.1-50wt%, but more preferably 1-30wt% relative to the total amount of cosmetic preparation. This crosslinking type organopolysiloxane is not particularly limited, examples of commercial products being KSG-15, KSG-16, KSG-18, KSG-21, KSG-31, KSG-32, KSG-33 and KSG-34 (Shin-Etsu Chemical Co.).

The cosmetic preparation of this invention may employ one, two or more silicone resins as component I) according to the purpose . This silicone resin is preferably an acrylic silicone resin of an acrylic/silicone graft or block copolymer. An acrylic silicone resin containing at least one moiety selected from among a pyrrolidone part, long-chain alkyl part, polyoxyalkylene part and fluoroalkyl part or anionic part such as a carboxylic acid, may also be used.

This silicone resin is preferably a silicone reticular compound expressed by MQ, MDQ, MT, MDT or MDTQ as a structural component. This M, D, T, Q respectively express an R₃Si0.5 unit, R₂SiO unit, RSiO_{1.5} unit or SiO₂ unit, and are commonly used in the silicone industry. Silicone reticular resins are commonly known as MQ resin, or MT and MDT resin. They not only may contain the part expressed by MDQ or MDTQ, but may also be sold as octamethylcyclotetrasiloxane solutions or the like. A silicone reticular compound having at least one moiety selected from a group comprising a pyrrolidone part, long-chain alkyl part, polyoxyalkylene part and fluoroalkyl part or amino part in the molecule, may also be used.

The blending amount when a silicone resin such as an acrylic silicone resin or silicone reticular compound which is component I) is used, is preferably 0.1-20wt%, but more preferably 1-10wt% relative to the total amount of cosmetic preparation.

To the present cosmetic preparation, the ingredients used in general cosmetic preparations, such as an oil-soluble gelling agent, clay mineral modified with organic compounds, resin, ultraviolet absorbent, moisture-holding agent, preservative, antimicrobial agent, perfume, salt, antioxidant, pH regulator, chelating agent, refrigerant, anti-inflammatory agent, skin beautifying component (skin whitener, cell activator, rough dry skin improver, blood circulation promoter, skin astringent or anti-seborrheicagent), vitamin, aminoacid, nucleic acid, hormone, clathrate compound and hair firming agent, can be added so far as they have no adverse influence on the effects of the present invention.

Examples of an oil-soluble gelling agent which can be added include metallic soaps, such as aluminum stearate, magnesium stearate and zinc myristate; amino acid derivatives, such as N-lauroyl-L-glutamic acid and α,γ-di-n-butylamine; dextrin fatty acid esters, such as dextrin palmitic acid ester, dextrin stearic acid ester and dextrin 2-ethylhexaminic acid palmitic acid ester; sucrose fatty acid esters, such as sucrose palmitic acid ester and sucrose stearic acid ester; benzylidene derivatives of sorbitol, such as monobenzylidene sorbitol and dibenzylidene sorbitol; and clay minerals modified with organic compounds, such as dimethylbenzyldodecyl ammonium montmorillonite clay and dimethyldioctadecyl ammonium montmorillonite clay.

Examples of an antiperspirant which can be added may be selected from among aluminum chlorohydrate, aluminum chloride, aluminumsesquichlorohydrate, zirconyl hydroxychloride, aluminum zirconium hydroxychloride and aluminum zirconium glycine complex. Examples of an ultraviolet absorbent which can be added include ultraviolet absorbents of benzoic acid type, such as p-aminobenzoic acid; those of anthranilic acid type, such as methyl anthranilate; those of salicylic acid type, such as methyl salicylate; those of succinic acid type, such as octyl p-methoxysuccinate; those of benzophenone type, such as 2,4-dihydroxybenzophenone; those of urocanic acid type, such as ethyl urocanate; and those of dibenzoylmethane type, such as 4-t-butyl-4'-methoxydibenzoylmethane. Examples of an ultraviolet absorption and scattering agent which can be added are powders which absorb and scatter ultraviolet light such as fine particle titanium oxide, fine particle iron-containing titanium oxide, fine particle zinc oxide, fine particle cerium oxide and their complexes.

Examples of a moisture-holding agent which can be added include glycerin, sorbitol, propylene glycol, dipropylene glycol, 1,3-butylene glycol, glucose, xylitol, maltitol, polyethylene glycol, hyaluromic acid, chondroitin sulfuric acid, pyrrolidone carboxylic acid, polyoxyethylene glycoside, and polyoxypropylene methylglycoside.

Examples of an antiseptic and preservative agent which can be added include alkyl p-hydroxybenzoates, benzoic acid, sodium benzoate, sorbic acid, potassium sorbate and phenoxyethanol; and those of an antimicrobial agent which can be added include benzoic acid, salicylic acid, carbolic acid, sorbic acid, alkyl p-hydroxybenzoates, p-chlorometacresol, hexachlorophene, benzalkonium chloride, chlorhexidine chloride, trichlorocarbanilide, photosensitizer and phenoxyethanol.

Examples of an antioxidant which can be added include tocopherol, butylhydroxyanisole, dibutylhydroxytoluene and phytic acid; those of a pH regulator which can be added include lactic acid, citric acid, glycolic acid, succinic acid, tartaric acid, dl-malic acid, potassium carbonate, sodium hydrogen carbonate and ammonium hydrogen carbonate; those of a chelating agent which can be added include alanine, sodium ethylenediaminetetraacetate, sodium polyphosphate, sodium metaphosphate and phosphoric acid; those of a refrigerant which can be added include L-menthol and camphor; and those of an anti-inflammatory agent which can added include allantoin, glycyrrhizin and salts thereof, glycyrrhetinic acid and stearyl glycyrrhetinate, tranexamic acid and azulene.

Examples of a skin-beautifying component which can be added include whitening agents, such as placenta extract, arbutin, glutathione and Yukinoshita extract; cell activators, such as royal jelly, photosensitizer, cholesterol derivatives and calf blood extract; rough dry skin improvers; blood circulation improvers, such as nonylic acid vanillyl amide, benzyl nicotinate, β-butoxyethyl nicotinate, capsaicin, zingerone, cantharis tincture, ichtammol, caffeine, tannic acid, α-borneol, tocopheryl nicotinate, inositol hexanicotinate, cyclandelate, cinnarizine, tolazoline, acetyl choline, verapamil, cepharanthin and γ-oryzanol; skin astringents, such as zinc oxide and tannic acid; and anti-seborrheic agents, such as sulfur and thianthol.

Examples of vitamins which can be added include vitamin A, such as vitamin A oil, retinol, retinyl acetate and retinyl palmitate; vitamin B, including vitamin B2 such as riboflavin, riboflavin butyrate and flavin adenine nucleotide, vitamin B6 such as pyridoxine hydrochloride, pyridoxine dioctanoate and pyridoxine tripalmitate, vitamin B12 and its derivatives, and vitamin B15 and its derivatives; vitamin C, such as L-ascorbic acid, L-ascorbic acid dipalmitic ester, sodium (L-ascorbic acid)-z-sulfate and dipotassium L-ascorbic acid diphosphate; vitamin D, such as ergocalciferol and cholecarciferol; vitamin E, such as α-tocopherol, β-tocopherol, γ-tocopherol, dl-α-tocopheryl acetate, dl-α-tocopheryl nicotinate and dl-α-tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; and biotin.

Examples of an amino acid which can be added include glycine, valine, leucine, isoleucine, serine, threonine, phenylalanine, arginine, lysine, aspartic acid, glutamic acid, cystine, cysteine, methionine, and tryptophan; those of a nucleic acid which can be added include deoxyribonucleic acid; and those of hormone which can be added include estradiol and ethenyl estradiol.

Examples of a hair firming polymer compound are amphoteric, anionic, cationic or nonionic polymer compounds, e.g., polyvinyl pyrrolidone-polymer compounds such as polyvinyl pyrrolidone and vinyl pyrrolidone/vinyl acetate copolymer; acidic vinyl ether polymer compounds such as methyl vinyl ether/maleic anhydride alkyl half ester copolymer; acidic polyvinyl acetate polymers such as vinyl acetate/crotonic acid copolymer; acidic acrylic polymer compounds such as (meth)acrylic acid/alkyl(meth)acrylate copolymer and (meth)acrylic acid/alkyl(meth)acrylate/alkyl acrylamide copolymer; and amphoteric acrylic polymer compounds such as N-methacryloylethyl-N,N-dimethyl ammonium-α-N-methyl carboxybetaine/alkyl(meth)acrylate copolymer, and hydroxypropyl(meth)acrylate/butylaminoethylmethacrylate/acryl ic acid octylamide copolymer. Also, naturally-occurring polymer compounds such as cellulose or its derivatives, keratin, and collagen or its derivatives, can also be used.

The term "cosmetic preparation" as used herein is intended to include skin care products, such as milky lotion, cream, face cleansing cream, packs, oily liquid, massage material, rinsing agent, deodorants, hand cream and lip cream; makeup products such as foundation, powder, liquid foundation, oily foundation, rouge, eye shadow, mascara, eyeliner, eyebrow makeup and lipstick; hairdressing products, such as shampoo, rinse, treatment and sets; antiperspirants; and ultraviolet defense cosmetic preparations such as suncut milky lotion or suncut cream.

Additionally, the present cosmetic preparation may have any form, including liquid, emulsion, cream, solid, paste, gel, powder, compress, layers, mousse, spray or stick.

This invention will now be described in more detail referring to specific examples, but this invention is not limited thereto. Unless otherwise specified, "%" refers to "wt%".

### Example 1

160.8 g of the methylhydrogenpolysiloxane expressed by the following formula (1), and 499.8 g of the methylvinylpolysiloxane expressed by the following formula (2) were introduced into a planetary mixer having an internal volume of approximately 51, then 0.1g of a 2% ethanol solution of chloroplatinic acid was added. Next, the internal temperature was raised to 70-80°C, and polymerization was performed for 2 hours to obtain a crosslinking type organopolysiloxane polymer. 30 wt parts of the obtained polymer and 30 wt parts of liquid paraffin were kneaded together to give composition (I).

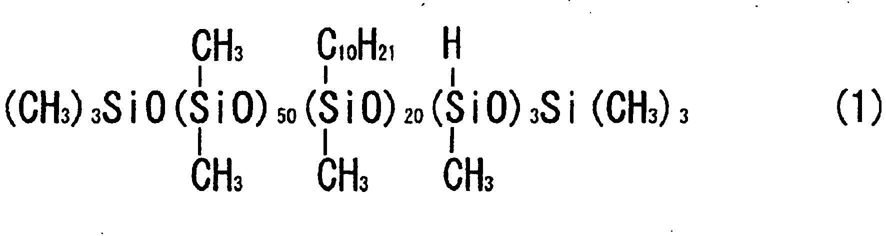

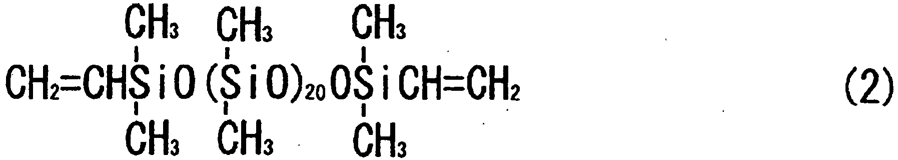

### Example 2

25 wt parts of the crosslinking type organopolysiloxane polymer obtained in Example 1 and 75 wt parts of isododecane were kneaded together to give composition (II).

### Example 3

30 wt parts of the crosslinking type organopolysiloxane polymer obtained in Example 1 and 70 wt parts of glyceryl isooctanoate were kneaded together to give composition (III).

### Example 4

30 wt parts of the crosslinking type organopolysiloxane polymer obtained in Example 1 and 70 wt parts of squalane were kneaded together to give composition (IV).

### Example 5

30 wt parts of the crosslinking type organopolysiloxane polymer obtained in Example 1, 50 wt parts of glyceryl isooctanoate and 20 wt parts of macadamia nut oil were kneaded together to give composition (V).

### Example 6

585.7g of the methylhydrogenpolysiloxane expressed by the following formula (3) and 53.8g of the methlyphenylvinyl polysiloxane expressed by the following formula (4) were blended as starting materials, then 0.1g of a 2% ethanol solution of chloroplatinic acid was added, and a crosslinking type organopolysiloxane polymer was obtained as in Example 1. 25 wt parts of this polymer and 75 wt parts of isononyl isononanoate were kneaded together to obtain composition (VI).

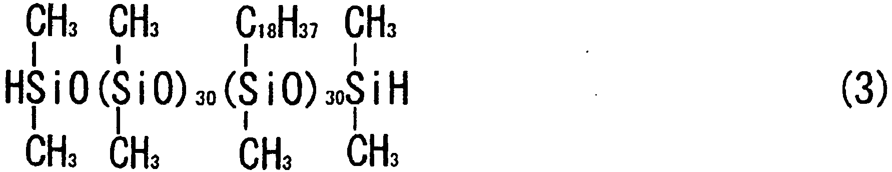

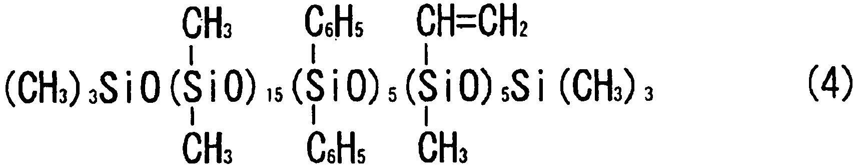

### Example 7: W/O type Emulsion

| (Component) | Weight (%) |
|---|---|
| 1. Composition (III) (Example 3) | 15.0 |
| 2. Dimethylpolysiloxane (6mm²/s (25°C)) | 12.0 |
| 3. Decamethylcyclopentasiloxane | 10.0 |
| 4. Glyceryl trioctanoate | 5.0 |
| 5. Polyether-modified silicone (Note 1) | 3.0 |
| 6. 1,3-butylene glycol | 5.0 |
| 7. Preservative | Suitable amount |
| 8. Perfume | Suitable amount |
| 9. Purified water | 50.0 |
| (Note 1) Shin-Etsu Chemical Co., Ltd.: KF-6017 (commercial name) | |

### (Manufacturing method)

### A: Components 1-5 were mixed uniformly.

### B: After mixing components 6-9, they were added to A and emulsified.

The W/O type emulsion thus obtained was not tacky and spread lightly. It also had good skin contact and setting qualities, and gave a gloss finish.

### Example 8: W/O type cream

| (Component) | Weight (%) |
|---|---|
| 1. Composition (I) (Example 1) | 6.0 |
| 2. Liquid paraffin | 13.5 |
| 3. Macadamia nut oil | 5.0 |
| 4. Alkyl/polyether co-modified silicone (Note 1) | 1.5 |
| 5. Sodium citrate | 0.2 |
| 6. Propylene glycol | 8.0 |
| 7. Glycerol | 3.0 |
| 8. Preservative | Suitable amount |
| 9.Perfume | Suitable amount |
| 10. Purified water | 62.8 |

| | |
|---|---|
| (Note 1) Shin-Etsu Chemical Co., Ltd.: KF-6026 (commercial name) (Manufacturing method) | |

### A: Components 1-4 were mixed.

### B: Components 5-10 were mixed, added to A, stirred and emulsified.

The W/O type cream thus obtained was not oily or, tacky, spread lightly and gave a fresh feeling. It also had good skin contact and setting qualities, and gave a gloss finish.

### Example 9: W/O type cream

| (Component) | Weight (%) |
|---|---|
| 1. Alkyl-modified crosslinking type polyether-modified silicone | |

| (Note 1) | 3.0 |
|---|---|
| 2. Composition (I) (Example 1) | 4.0 |
| 3. Liquid paraffin | 13.5 |
| 4. Macadamia nut oil | 5.0 |
| 5. Alkyl/polyether co-modified silicone (Note 2) | 0.5 |
| 6. Hybrid silicone composite powder (Note 3) | 3.0 |
| 7. Sodium citrate | 0.2 |
| 8. Propylene glycol | 8.0 |
| 9. Glycerol | 3.0 |
| 10. Preservative | Suitable amount |
| 11. Perfume | Suitable amount |
| 12. Purified water | 59.8 |

| | |
|---|---|
| (Note 1) Shin-Etsu Chemical Co., Ltd.: KSG-31 (commercial name) (Note 2) Shin-Etsu Chemical Co., Ltd.: KF-6026 (commercial name) (Note 3) Shin-Etsu Chemical Co., Ltd.: KSP-100 (commercial name) | |

### (Manufacturing method)

### A: Components 1-6 were mixed.

### B: Components 7-12 were mixed, added to A, stirred and emulsified.

The W/O type cream thus obtained was not oily or tacky, and spread lightly. It also had good skin contact and setting qualities, and gave a matt finish.

### Example 10: O/W type cream

| (Component) | Weight (%) |
|---|---|
| 1. Composition (V) (Example 5) | 8.0 |
| 2. Crosslinked methyl phenyl polysiloxane (Note 1) | 2.0 |
| 3. Isotridecyl isononanoate | 5.0 |
| 4. Dipropylene glycol | 7.0 |
| 5. Glycerol | 5.0 |
| 6. Methyl cellulose (2% aqueous solution) (Note 2) | 7.0 |
| 7. Polyacrylamide emulsifier (Note 3) | 2.0 |
| 8. Guanine | 1.0 |
| 9. Preservative | Suitable amount |
| 10. Perfume | Suitable amount |
| 11. Purified water | 63.0 |

| | |
|---|---|
| (Note 1) Shin-Etsu Chemical Co., Ltd.: KSG-18 (Note 2) Shin-Etsu Chemical Co., Ltd.: Metrose SM-4000 (Note 3) SEPIC: Sepigel 305 | |

### (Manufacturing method)

### A: Components 4-11 were mixed.

### B: Components 1-3 were mixed, then A was added, and stirred and emulsified.

The O/W type cream thus obtained was fine, spread lightly, was not tacky or oily, was moist and fresh, and gave a clean feeling. The cosmetic preparation lasted very well, showed no change with temperature or time, and had excellent stability.

### Example 11: W/O type cream

| (Component) | Weight (%) |
|---|---|
| 1. Composition (III) (Example 3) | 2.0 |
| 2. Dimethylpolysiloxane (6mm²/s (25°C)) | 10.0 |
| 3. Crosslinking type polyether-modified silicone | (Note 1) 5.0 |
| 4. Polyether-modified silicone (Note 2) | 0.5 |
| 5. Dipropylene glycol | 10.0 |
| 6. Sodium citrate | 0.2 |
| 7. Ethanol | 5.0 |
| 8. Preservative | Suitable amount |
| 9. Perfume | Suitable amount |
| 10. Purified water | 67.3 |

| | |
|---|---|
| (Note 1) Shin-Etsu Chemical Co., Ltd.: KSG-21 (commercial name) (Note 2) Shin-Etsu Chemical Co., Ltd.: KF-6017 (commercial name) | |

### (Manufacturing method)

### A: Components 1-4 were mixed.

### B: Components 5-10 were mixed and dissolved, added to A, stirred and emulsified.

The W/O type cream thus obtained was moist and fresh, not oily or tacky, spread lightly, and gave a fresh, clean feeling. It had good skin contact and setting qualities, and gave a matt finish.

### Example 12: W/O type makeup foundation

| (Component) | Weight (%) |
|---|---|
| 1. Crosslinked polyether-modified silicone (Note 1) | 4.0 |
| 2. Crosslinking type dimethylpolysiloxane (Note 2) | 1.0 |
| 3. Composition (II) (Example 2) | 1.0 |
| 4. Polyether-modified silicone (Note 3) | 0.5 |
| 5. Dimethylpolysiloxane (6mm²/s (25°C)) | 6.0 |
| 6.Dimethylpolysiloxane (20mm²/s (25°C)) | 2.0 |
| 7. Decamethylcyclopentasiloxane | 3.0 |
| 8. Titanium oxide/cyclopentasiloxane dispersion (Note 4) | 10.0 |
| 9. Dipropylene glycol | 5.0 |
| 10. Sodium citrate | 0.2 |
| 11. Methyl cellulose (2% aqueous solution) (Note 5) | 2.5 |
| 12. Ethanol | 3.0 |
| 13. Preservative | Suitable amount |
| 14. Perfume | Suitable amount |
| 15. Purified water | 61.8 |

| | |
|---|---|
| (Note 1) Shin-Etsu Chemical Co., Ltd.: KSG-21 (commercial name) (Note 2) Shin-Etsu Chemical Co., Ltd.: KSG-15 (commercial name) (Note 3) Shin-Etsu Chemical Co., Ltd.: KF-6017 (commercial name) (Note 4) Shin-Etsu Chemical Co., Ltd.: SPD-T1S (commercial name) (Note 5) Shin-Etsu Chemical Co., Ltd.: Metrose 65-SH4000 (commercial name) | |

### (Manufacturing method)

### A: Components 1-8 were mixed.

### B: Components 9-15 were mixed and dissolved, added to A, stirred and emulsified.

The W/O type makeup foundation thus obtained was not oily or tacky, spread lightly, and gave a fresh, clean feeling. It also had good skin contact and setting qualities, and gave a matt finish. Moreover, it had an ultraviolet cut effect and lasted well in cosmetics.

### Example 13: O/W type cream

| (Component) | Weight (%) |
|---|---|
| 1. Composition (II) (Example 2) | 2.0 |
| 2. Crosslinking type dimethylpolysiloxane (Note 1) | 15.0 |
| 3. Decamethylcyclopentasiloxane | 10.0 |
| 4. Dimethylpolysiloxane (6mm²/s (25°C)) | 18.0 |
| 5. Polyether-modified silicone (Note 2) | 0.7 |
| 6. Propylene glycol | 3.0 |
| 7. Polyacrylamide mixture (Note 3) | 0.8 |
| 8. Xanthan gum (2% aqueous solution) | 8.0 |
| 9. Preservative | Suitable amount |
| 10. Perfume | Suitable amount |
| 11. Purified water | 42.5 |

| | |
|---|---|
| (Note 1) Shin-Etsu Chemical Co., Ltd.: KSG-16 (commercial name) (Note 2) Shin-Etsu Chemical Co., Ltd.: KF-6011 (commercial name) (Note 3) SEPIC: Sepigel 305 (commercial name) | |

### (Manufacturing method)

### A: Components 1-4 were mixed.

### B: Components 5-11 were mixed and dissolved.

### C: A was added to B, stirred and emulsified.

The O/W type cream thus obtained was fine, spread lightly, was not tacky or oily, was moist and fresh, and gave a clean feeling. The cosmetic preparation lasted very well, showed no change with temperature or time, and had excellent stability

### Example 14: Lipstick

| (Component) | Weight (%) |
|---|---|
| 1. Polyethylene wax | 12.0 |
| 2.Microcrystallinewax | 4.0 |
| 3.Polybutene | 5.0 |
| 4.Acrylate/dimethylsilicone copolymer (Note 1) | 12.0 |
| 5. Composition (I) (Example 1) | 7.0 |
| 6. Cetyl octanoate | 20.0 |
| 7. Cane sugar fatty acid ester | 3.0 |
| 8. Glyceryltryisostearate | 37.0 |
| 9. Pigment | Suitable amount |
| 10. Preservative | Suitable amount |
| 11. Perfume | Suitable amount |

| | |
|---|---|
| (Note 1) Shin-Etsu Chemical Co., Ltd.: KP-561 | |

### (Manufacturing method)

### A: Components 1-7 and part of Component 8 were heated, mixed and dissolved.

### B: Component 9 and the remainder of Component 8 were uniformly mixed, added to A, and homogenized.

The lipstick thus obtained spread lightly, was not oily or powdery, and gave a clean feeling. It had good water resistance and water repellence, lasted well, and had excellent stability.

### Example 15: Powder foundation

| (Component) | Weight (%) |
|---|---|
| 1. Vaseline | 2.5 |
| 2. Squalane | 3.0 |
| 3. Composition (IV) (Example 4) | 0.5 |
| 4. Glyceryl trioctanoate | 2.0 |
| 5. Silicone-treated mica | 40.0 |
| 6. Silicone-treated talc | Remainder |
| 7. Silicone-treated titanium oxide | 10.0 |
| 8. Silicone-treated particulate titanium oxide | 5.0 |
| 9. Silicone-treated barium sulfate | 10.0 |
| 10. Pigment | Suitable amount |
| 11. Fluorine-modified hybrid silicone composite powder (Note 1) | 2.0 |
| 12. Silicone powder (Note 2) | 2.5 |
| 13. Preservative | Suitable amount |
| 14. Perfume | Suitable amount |

| | |
|---|---|
| (Note 1) Shin-Etsu Chemical Co., Ltd.: KSP-200 (commercial name) (Note 2) Shin-Etsu Chemical Co., Ltd.: KMP-590 (commercial name) | |

### (Manufacturing method)

### A: Components 4-13 were mixed, and homogenized.

### B: Components 1-3 were mixed uniformly, added toA, and homogenized. C: Component 14 was added to B, and press molded into a mold to obtain a foundation.

The powder foundation thus obtained was not tacky, and spread lightly. It had good skin contact and setting qualities, and gave a gloss finish.

### Example 16: Cream foundation

| (Component) | Weight (%) |
|---|---|
| 1. Crosslinking type polyether-modified silicone | |
| (Note 1) | 4.0 |
| 2. Composition (VI) (Example 6) | 1.5 |
| 3. Glyceryl trioctanoate | 4.0 |
| 4. Dimethylpolysiloxane (6mm²/s (25°C)) | 5.0 |
| 5. Decamethylcyclopentasiloxane | 5.0 |
| 6. Fluorine-modified hybrid silicone composite powder (Note 2) | 2.5 |
| 7. Pigment | 8.0 |
| 8. Acrylic silicone resin (Note 3) | 5.0 |
| 9. Dipropylene glycol | 5.0 |
| 10. Sodium citrate | 0.2 |
| 11. Preservatiev | Suitable amount |
| 12. Perfume | Suitable amount |
| 13. Purified water | 59.3 |

| | |
|---|---|
| (Note 1) Shin-Etsu Chemical Co., Ltd.: KSG-21 (commercial name) (Note 2) Shin-Etsu Chemical Co., Ltd.: KSP-200 (commercial name) (Note 3) Shin-Etsu Chemical Co., Ltd.: KP-545 (commercial name) | |

### (Manufacturing method)

### A: Components 1-6 were mixed.

### B: Components 9-13 were mixed and dissolved, added to A, stirred and emulsified.

### C: Components 7-8 were mixed, added to B, and homogenized.

The cream foundation thus obtained was not tacky and spread lightly. It had good skin contact and setting qualities, and gave a matt finish.

### Example 17: W/O compact foundation

| (Component) | Weight (%) |
|---|---|
| 1. Ceresin | 5.5 |
| 2. Microcrystalline wax | 1.0 |
| 3. Liquid paraffin | 3.0 |
| 4. Composition (I) (Example 1) | 1.0 |
| 5. Dicapric acid polypropylene glycol | 3.0 |
| 6. Alkyl/polyether co-modified silicone (Note 1) | 1.0 |
| 7. Alkyl-modified crosslinking type polyether-modified silicone (Note 2) | 8.0 |
| 8. Dimethylpolysiloxane (6mm²/s (25°C)) | 15.5 |
| 9. Oil-treated titanium oxide | 10.0 |
| 10.Pigment | Suitable amount |
| 11. Lecithin | 0.3 |
| 12. Monooleic acid polyoxyethylene sorbitan | 0.5 |
| 13. Dipropylene glycol | 8.0 |
| 14. Sodium citrate | 0.2 |
| 15. Purified water | Remainder |

| | |
|---|---|
| (Note 1) Shin-Etsu Chemical Co., Ltd.: KF-6026 (commercial name) (Note 2) Shin-Etsu Chemical Co., Ltd.: KSG-33 (commercial name) | |

### (Manufacturing method)

### A: Components 1-8 were heated and mixed.

### B: Components 9-13 were mixed uniformly.

C: Components 14-15 were mixed, B was added, mixed uniformly and heated.

### D: C was added to A and emulsified.

Although this W/O type compact foundation contained a large amount of oil, it was not oily or tacky, spread lightly and gave a clean feeling. It also had good skin contact and setting qualities, and lasted very well in cosmetic preparations.

### Example 18: Powder eyebrow

| (Component) | Weight (%) |
|---|---|
| 1. Vaseline | 2.5 |
| 2. Dimethylpolysiloxane (6mm²/s (25°C)) | 1.5 |
| 3. Composition (III) (Example 3) | 0.5 |
| 4. Glyceryl trioctanoate | 4.0 |
| 5. Silicone-treated mica | 40.0 |
| 6. Silicone-treated talc | Remainder |
| 7. Silicone-treated titanium oxide | 10.0 |
| 8.Silicone-treated barium sulfate | 15.0 |
| 9. Silicone-treated pigment | Suitable amount |
| 10. Hybrid silicone composite powder (Note 1) | 1.5 |
| 11. Spherical polymethylsilsesquioxane fine particles (Note 2) | 2.5 |
| 12. Preservative | Suitable amount |
| 13. Perfume | Suitable amount |

| | |
|---|---|
| (Note 1) Shin-Etsu Chemical Co., Ltd.: KSP-100 (commercial name) (Note 2) Shin-Etsu Chemical Co., Ltd.: KMP-590 (commercial name) | |

### (Manufacturing method)

### A: Components 5-12 were mixed and homogenized.

### B: Components 1-4 were mixed uniformly, added to A, and the mixture was homogenized.

### C: Component 13 was added to B, and the mixture was pressed in a mold to obtain a powder eyebrow.

The eyebrow thus obtained was not tacky and spread lightly. It had good skin contact and setting qualities, gave a gloss finish, and lasted well in cosmetic preparations.

### Example 19: Conditioning mousse

| (Component) | Weight (%) |
|---|---|
| 1. Composition (III) (Example 3) | 0.5 |
| 2. Dimethylpolysiloxane (6mm²/s (25°C)) | 2.0 |
| 3. Crosslinking type dimethylpolysiloxane (Note 1) | 0.5 |
| 4. Glyceryl trioctanoate | 1.5 |
| 5. Glycerol | 3.0 |
| 6. Stearyldimethylbenzylammonium chloride | 0.5 |
| 7. Polyoxyethylene-hardened castor oil | 0.5 |
| 8. Ethanol | 7.0 |
| 9. Preservative | Suitable amount |
| 10. Perfume | Suitable amount |
| 11. Purified water | Remainder |
| 12. liquefied petroleum gas | 5.0 |

| | |
|---|---|
| (Note 1) Shin-Etsu Chemical Co., Ltd. KSG-16 (commercial name) | |

### (Manufacturing method)

### A: Components 1-4 were heated and dissolved.

### B: Components 5-9 and 11 were mixed uniformly and dissolved.

### C: B was added to A, emulsified, cooled, and Component 10 was added. D: An aerosol can was filled with C, and a conditioning mousse was obtained.

The conditioning mousse thus obtained was moist, flexible, very smooth, non-oily and left a good feeling. It also had good skin contact and setting qualities, and gave a matt finish.

### Example 20: Roll-on type antiperspirant

| (Component) | Weight (%) |
|---|---|
| 1. Composition (II) (Example 2) | 5.0 |
| 2.Crosslinking type dimethylpolysiloxane (Note 1) | 20.0 |
| 3. Dimethylpolysiloxane (6mm²/s (25°C)) | 10.0 |
| 4. Crosslinking type dimethylpolysiloxane (Note 2) | 15.0 |
| 5. Decamethylcyclopentasiloxane | 30.0 |
| 6. Aluminum zirconium tetrachlorohydrate | 20.0 |
| 7. Perfume | Suitable amount |

| | |
|---|---|
| (Note 1) Shin-Etsu Chemical Co., Ltd. KSG-21 (commercial name) (Note 2) Shin-Etsu Chemical Co., Ltd. KSG-15 (commercial name) | |

### (Manufacturing method)

### A: Components 1-5 were mixed.

### B: Components 6 and 7 were added to A, and dispersed uniformly.

The roll-on antiperspirant thus obtained spread lightly, gave a cool, fresh and clean feeling without tackiness or oiliness, and showed no change with temperature or time. It was moreover easy to use, and very stable.

### Example 21: W/O type antiperspirant

| (Component) | Weight (%) |
|---|---|
| 1. Composition (III) (Example 3) | 2.0 |
| 2. Crosslinking type polyether-modified silicone (Note 1) | 7.0 |
| 3. Decamethylcyclopentasiloxane | 7.0 |
| 4. Glyceryl trioctanoate | 8.0 |
| 5. 1,3-butylene glycol | 5.0 |
| 6. Sodium citrate | 0.2 |
| 7. Aluminum chlorohydrate | 20.0 |
| 8. Perfume | Suitable amount |
| 9. Purified water | 50.8 |

### (Manufacturing method)

### A: Components 1-4 were mixed.

### B: Components 5-6 and 9 were mixed, and Components 7 and 8 were added and dissolved.

### C: B was added to A, stirred and emulsified.

The W/O antiperspirant thus obtained spread lightly, gave a cool, fresh and clean feeling, and was not tacky or oily. It also showed no change with temperature or time, was very easy to use and very stable.

### Example 22: W/O type UV cut cream

| (Component) | Weight (%) |
|---|---|
| 1. Silicone-treated zinc oxide | 20.0 |
| 2. Acrylate/dimethylsilicone copolymer (Note 1) | 12.0 |
| 3. Decamethylcyclopentasiloxane | 20.0 |
| 4. Glyceryl trioctanoate | 3.0 |
| 5. Composition (III) (Example 3) | 2.0 |
| 6. Crosslinking type polyether-modified silicone (Note 2) | 5.0 |
| 7. Polyether-modified silicone (Note 3) | 1.0 |
| 8. Alkyl/polyether co-modified silicone (Note 4) | 1.0 |
| 9. Octyl methoxycinnamate | 6.0 |
| 10. Sodium citrate | 0.2 |
| 11. Dipropylene glycol | 3.0 |
| 12. Preservative | Suitable amount |
| 13. Perfume | Suitable amount |
| 14. Purified water | 26.8 |

| | |
|---|---|
| (Note 1) Shin-Etsu Chemical Co., Ltd.: KP-545 (commercial name) (Note 2) Shin-Etsu Chemical Co., Ltd.: KSG-21 (commercial name) (Note 3) Shin-Etsu Chemical Co., Ltd.: KF-6017 (commercial name) (Note 4) Shin-Etsu Chemical Co., Ltd.: KF-6026 (commercial name) | |

### (Manufacturing method)

### A: Part of Component 3 and Components 4-9 were mixed.

### B: Components 10-12 and 14 were mixed, added to A, stirred and emulsified.

### C: Components 1, 2 and the remainder of Component 3 were mixed and dispersed,

### Component 13 was added to B, and the mixture homogenized.

The W/O type UV cut cream thus obtained spread lightly, gave a fresh feeling without tackiness or oiliness, was transparent, and lasted well in cosmetic preparations. It showed no change with temperature or time, was very easy to use and very stable.

### Example 23: W/O type UV cut milky lotion

| (Component) | Weight (%) |
|---|---|
| 1. Dimethylpolysiloxane (6mm²/s (25°C)) | 5.0 |
| 2. Crosslinking type polyether-modified silicone (Note 1) | 5.0 |
| 3. Glyceryl trioctanoate | 2.0 |
| 4. Composition (II) (Example 2) | 1.0 |
| 5. Polyether-modified silicone (Note 2) | 1.0 |
| 6. Titanium oxide/decamethylcyclopentasiloxane dispersion (Note 3) | 30.0 |
| 7. Zinc oxide/decamethylcyclopentasiloxane dispersion (Note 4) | 30.0 |
| 8. Dipropylene glycol | 3.0 |
| 9. Sodium citrate | 0.2 |
| 10. Preservative | Suitable amount |
| 11. Perfume | Suitable amount |
| 12. Purified water | 22.8 |

| | |
|---|---|
| (Note 1) Shin-Etsu Chemical Co., Ltd.: KSG-21 (commercial name) (Note 2) Shin-Etsu Chemical Co., Ltd.: KF-6017 (commercial name) (Note 3) Shin-Etsu Chemical Co., Ltd. SPD-T1S (commercial name) (Note 4) Shin-Etsu Chemical Co., Ltd.: SPD-Z1S (commercial name) | |

### (Manufacturing method)

### A: Components 1-5 were mixed.

### B : Components 8-10 and Component 12 were mixed and dissolved, added to A, stirred and emulsified.

### C: Components 6, 7 and 11 were added to B, and homogenized.

The W/O type UV cut milky lotion thus obtained spread lightly, gave a fresh feeling without tackiness or oiliness, was transparent, and lasted well in cosmetic preparations. It showed no change with temperature or time, was very easy to use and very stable.

### Example 24: O/W type UV cut cream

| (Component) | Weight (%) |
|---|---|
| 1. Crosslinked organopolysiloxane (Note 1) | 5.0 |
| 2.Cetylisooctanoate | 5.0 |
| 3. Composition (VI) (Example 6) | 1.0 |
| 4. Titanium oxide/decamethylcyclopentasiloxane dispersion (Note 2) | 15.0 |
| 5. Polyether-modified silicone (Note 3) | 1.0 |
| 6. Polyether-modified silicone (Note 4) | 1.0 |
| 7. Acrylic acid amide mixture (Note 5) | 2.0 |
| 8. Propylene glycol | 5.0 |
| 9. Methylcellulose (2% aqueous solution) (Note 6) | 5.0 |
| 10. Preservative | Suitable amount |
| 11. Perfume | Suitable amount |
| 12. Purified water | 60.0 |

| | |
|---|---|
| (Note 1) Shin-Etsu Chemical Co., Ltd.: KSG-18 (commercial name) (Note 2) Shin-Etsu Chemical Co., Ltd.: SPD-T1S (commercial name) (Note 3) Shin-Etsu Chemical Co., Ltd.: KF-6027 (commercial name) (Note 4) Shin-Etsu Chemical Co., Ltd.: KF-6011 (commercial name) (Note 5) Seppic: Sepigel 305 (commercial name) (Note 6) Shin-EtsuChemical Co., Ltd.: Metrose SM-4000 (commercial name) | |

### (Manufacturing method)

### A: Components 5-8, 10 and 12 were mixed.

### B: Components 1-3 were mixed, added to A, stirred and emulsified. C: Component 4 was added to B, Components 9 and 11 were added, and homogenized.

The W/O type UV cut cream thus obtained spread lightly, gave a fresh feeling without tackiness or oiliness, was transparent, and lasted well in cosmetic preparations. It showed no change with temperature or time, was very easy to use and very stable.

### Example 25: Nonaqueous emulsion

| (Component) | Weight (%) |
|---|---|
| 1. Crosslinking type dimethylpolysiloxane (Note 1) | 30.0 |
| 2. Decamethylcyclopentasiloxane | 15.0 |
| 3. Dimethylpolysiloxane (6mm²/s) | 7.0 |
| 4. Composition (III) (Example 3) | 3.0 |
| 5. Dimethyldistearylammonium hectorite | 2.0 |
| 6. Sodium chloride | 0.1 |
| 7.1,3-butyleneglycol | 42.9 |

| | |
|---|---|
| (Note 1) Crosslinking type dimethyl polysiloxane:KSG15 (Shin-Etsu Chemical Co., Ltd.) | |

### (Manufacturing method)

### A: Components 1-5 were mixed uniformly.

### B: Components 6 and 7 were mixed.

### C: B was added to A and emulsified uniformly.

The nonaqueous emulsion thus obtained spread lightly without being tacky or oily, left the skin feeling soft, and was very stable.

### Example 26: W/O/W type cream

| (Component) | Weight (%) |
|---|---|
| 1. Crosslinking type dimethylpolysiloxane (Note 1) | 5.0 |
| 2. Cetyl isooctanoate | 5.0 |
| 3. Composition (III) (Example 3) | 1.0 |
| 4. Decamethylcyclopentasiloxane | 5.0 |
| 5. Dioleic acid methyl glucose | 1.5 |
| 6. Isohexadecane | 3.5 |
| 7. Magnesium sulfate | 0.5 |
| 8. Propylene glycol | 5.0 |
| 9. Purified water | 39.5 |
| 10. Cetylalcohol | 1.0 |
| 11. PEG-10 soya sterol | 2.0 |
| 12. Preservative | Suitable amount |
| 13. Perfume | Suitable amount |
| 14. Purified water | 31.0 |

### (Manufacturing method)

### A: Components 7-9 were mixed.

### B: Components 1-6 were mixed, added to A, stirred and emulsified. C: Components 10-12 and 14 were mixed, B was added with stirring, and emulsified.

### D: Component 13 was added to C, and homogenized.

The W/O/W type cream thus obtained spread lightly, gave a fresh feeling without tackiness or oiliness, was transparent, and lasted well in cosmetic preparations. It showed no change with temperature or time, was very easy to use and very stable.

### Example 27: O/W/O type facial liquid foundation

| (Component) | Weight (%) |
|---|---|
| 1. Crosslinking type organopolysiloxane (Note 1) | 5.0 |
| 2. Decanoic acid propylene glycol | 5.0 |
| 3. Isopropylmyristate | 5.0 |
| 4. Pigment | 10.0 |
| 5. Egg yolk-derived hydrogenated phospholipid | 1.0 |
| 6. Glycerol | 2.0 |
| 7.1,3-butyleneglycol | 10.0 |
| 8. Preservative | Suitable amount |
| 9. Purified water | 52.0 |
| 10. Squalane | 3.0 |
| 11. Composition (IV) (Example 4) | 2.0 |
| 12. Cetyl alcohol | 5.0 |
| 13. Perfume | Suitable amount |

| | |
|---|---|
| (Note 1) Shin-Etsu Chemical Co.. Ltd.: KSG-21 (commercial name) | |

### (Manufacturing method)

### A: Components 1-3 were mixed uniformly.

### B: Components 4-9 were heated and mixed.

### C: Components 10-13 were heated and mixed.

### D: B was stirred, C was added, emulsified, and cooled.

### D: A was stirred, D was added, and emulsified,.

The O/W/O type liquid foundation thus obtained spread lightly, gave a fresh feeling without tackiness or oiliness, was transparent, and lasted well in cosmetic preparations. It showed no change with temperature or time, was very easy to use and very stable.

The cosmetic preparation obtained by blending a composition containing the crosslinking type organopolysiloxane polymer of this invention with an oil other than a silicone oil, spreads lightly and gives a fresh feeling. When it is applied, it is not tacky or heavy, and leaves the skin feeling smooth. Therefore, this cosmetic preparation has excellent properties when used. Upon application, it imparts flexibility and smoothness and has an emollient effect without impairing moderate transpiration. It imparts properties widely ranging from natural gloss to a matt feeling. The cosmetic preparation further has satisfactory long-term stability.

### Industrial Field of Application

The cosmetic preparation of this invention is exceedingly useful. It spreads lightly and gives a fresh feeling, and upon application, it imparts flexibility and smoothness and has an emollient effect. It imparts properties widely ranging from natural gloss to a matt feeling, and also has satisfactory long-term stability. The composition of this invention is also one of the starting materials of the cosmetic preparation, and it therefore has considerable industrial utility.

## Claims

1. A pasty composition comprising a crosslinking type organopolysiloxane which swells up by containing at least its own weight of an oil other than a silicone oil, and an oil other than a silicone oil,
**characterized in that** said crosslinking type organopolysiloxane polymer is a crosslinking type organopolysiloxane polymer obtained by addition polymerization of the organohydrogenpolysiloxane expressed by the following (1) with the vinyl group-containing organopolysiloxane expressed by the following (2).
(1): organohydrogen polysiloxane comprising at least one type of structural unit selected from groups comprising a SiO₂ unit, HSiO_{1.5} unit, RSiO_{1.5} unit, RHSiO unit, R₂SiO unit, R₃SiO_{0.5} unit and R₂HSiO_{0.5} unit, wherein, R is a monofunctional hydrocarbon group having 1-30 carbon atoms which may be substituted or unsubstituted, but R cannot be an aliphatic unsaturated group, and comprising on average 1.5 or more hydrogen atoms bonded to a silicon atom in the molecule.
(2) : organopolysiloxane comprising at least one type of structural unit selected from among a SiO₂ unit, (CH₂=CH)SiO_{1.5} unit, RSiO_{1.5} unit, R(CH₂=CH)SiO unit, R₂SiO unit, R₃SiO_{0.5} unit and R₂(CH₂=CH)SiO_{0.5} unit, wherein, R is identical to that of the aforesaid (1), and comprising on average 1.5 or more vinyl groups bonded to a silicon atom in the molecule,.
wherein, for at least one of the R in the aforesaid general equations (1) and (2), 30 mol% or more is a methyl group, and 5-50 mol% is a hydrocarbon group having 10-22 carbon atoms.
Herein, the organopolysiloxanes expressed by (1) and (2) are selected so that the total content of at least one of the hydrogen atoms bonded to silicon atom and the vinyl groups bonded to silicon atom is less than 20 mol% of the sum of all organic groups and hydrogen atoms bonded to silicon atom.

2. The composition according to claim 1, wherein said oil other than a silicone oil is a hydrocarbon oil.

3. The composition according to claim 2, wherein said hydrocarbon oil is a branched hydrocarbon oil.

4. The composition according to claim 2 or 3, wherein said hydrocarbon oil is a volatile hydrocarbon oil.

5. The composition according to any of claims 1-4, wherein said oil other than a silicone oil is an ester oil.

6. The composition according to any of claims 1-4, wherein said oil other than a silicone oil is a glyceride oil.

7. The composition according to any of claims 1-4, wherein said oil other than a silicone oil is natural animal or vegetable oil and semi-synthetic oil.

8. A cosmetic preparation comprising the composition according to any of claims 1-7 as component A.

9. The cosmetic preparation according to claim 8, containing an oil other than the oil which is contained in component A), as component B).

10. The cosmetic preparation according to claim 9, wherein at least part of the oil which is component B) is a liquid at ordinary temperature.

11. The cosmetic preparation according to claim 9 or 10,
wherein at least part of the oil which is component B) is a straight-chain, branched or cyclic silicon oil expressed by R₁ₐSiO_{(4-a)/2}, wherein R₁ is a hydrogen atom or an alkyl group having 1-30 carbon atoms, aryl group, aralkyl group or fluorine-substituted alkyl group, and a is a positive number within the range of 1.5≤a≤2.5.

12. The cosmetic preparation according to any of claims 9-11, wherein at least part of the oil which is component B) is an oil having a fluorine group or an amino group.

13. The cosmetic preparation according to any of claims 8-12, containing water as component C).

14. The cosmetic preparation according to any of claims 8 to 13, containing a compound having an alcoholic hydroxyl group in its molecular structure as component D).

15. The cosmetic preparation according to claim 14, wherein the compound having an alcoholic hydroxyl group in its molecular structure as component D), is a water-soluble monohydric alcohol and/or water-soluble polyhydric alcohol.

16. The cosmetic preparation according to any of claims 8-15, containing a water-soluble or water-swelling polymer as component E).

17. The cosmetic preparation according to claims 8-16, containing a powder and/or colorant as component F).

18. The cosmetic preparation according to claim 17, wherein at least part of said powder and/or colorant which is component F), is a crosslinking type silicone fine powder having a structure wherein dimethyl silicone is crosslinked, polymethylsilsesquioxane fine powder, hydrophobic silica or a composite fine powder wherein a spherical silicone rubber surface is coated with polymethylsilsesquioxane particles.

19. The cosmetic preparation according to claim 17 or 18,
wherein at least part of the powder and/or colorant which is component F) is a powder and/or colorant having a fluorine group.

20. The cosmetic preparation according to any of claims 8-19, containing a surfactant as component G).

21. The cosmetic preparation according to claim 20, wherein the surfactant which is component G) is a straight-chain or branched silicone having a polyoxyalkylene chain in the molecule.

22. The cosmetic preparation according to claim 20 or 21,
wherein the HLB of the surfactant which is component G) is 2-8.

23. The cosmetic preparation according to any of claims 8-22, containing a crosslinking type organopolysiloxane which swells up with at least its own weight of a low viscosity silicone oil having a viscosity of 0.65mm²/s (25°C)-100.0mm²/s (25°C) as component H).

24. The cosmetic preparation according to claim 23, wherein the crosslinking type organopolysiloxane which is component H), is a crosslinking type organopolysiloxane containing at least one part selected from among a polyoxyalkylene part, alkyl part, alkenyl part, aryl part and fluoroalkyl part in the crosslinked molecule.

25. The cosmetic preparation according to any of claims 8-24, containing a silicone resin as component I).

26. The cosmetic preparation according to claim 25, wherein the silicone resin which is component I) is an acrylic silicone resin.

27. The cosmetic preparation according to claim 26, wherein the silicone resin which is component I) is an acrylic silicone resin containing at least one part selected from among a pyrrolidone part, long-chain alkyl part, polyoxyalkylene part, fluoroalkyl part, and anionic part such as a carboxylic acid in the molecule.

28. The cosmetic preparation according to claim 25, wherein the silicone resin which is component I) is a silicone reticular compound expressed by MQ, MDQ, MT, MDT or MDTQ as a structural component.

29. The cosmetic preparation according to claim 25 or 28,
wherein the silicone resin which is component I) is a silicone reticular compound containing at least one part selected from among a pyrrolidone part, long-chain alkyl part, polyoxyalkylene part, fluoroalkyl part and amino part in the molecule.

## Patentansprüche

1. Pastöse Zusammensetzung, umfassend ein Organopolysiloxan vom Vernetzungstyp, das dadurch aufquellen kann, dass es mindestens sein Eigengewicht an einem Öl, das von einem Silikonöl verschieden ist, aufnimmt, und ein Öl, das von einem Silikonöl verschieden ist,
**dadurch gekennzeichnet, dass** es sich bei dem Organopolysiloxanpolymer vom Vernetzungstyp um ein durch Additionspolymerisation des Organohydropolysiloxans, das durch das folgende (1) dargestellt ist, mit dem vinylgruppenhaltigen Organopolysiloxan, das durch das folgende (2) dargestellt ist, erhaltenes Organopolysiloxanpolymer vom Vernetzungstyp handelt:
(1): Organohydrogenpolysiloxan, umfassend mindestens einen Typ von Struktureinheit, der unter Gruppen umfassend eine SiO₂-Einheit, HSiO_{1,5}-Einheit, RSiO_{1,5}-Einheit, RHSiO-Einheit, R₂SiO-Einheit, R₃SiO_{0,5}-Einheit und R₂HSiO_{0,5}-Einheit ausgewählt ist, wobei R für eine monofunktionelle Kohlenwasserstoffgruppe mit 1-30 Kohlenstoffatomen steht, die substituiert oder unsubstituiert sein kann, aber R nicht für eine aliphatische ungesättigte Gruppe stehen kann, und durchschnittlich 1,5 oder mehr an ein Siliciumatom gebundene Wasserstoffatome im Molekül;
(2) Organopolysiloxan, umfassend mindestens einen Typ von Struktureinheit, der unter einer SiO₂-Einheit, (CH₂=CH)SiO_{1,5}-Einheit, RSiO_{1,5}-Einheit, R(CH₂=CH)SiO-Einheit, R₂SiO-Einheit, R₃SiO_{0,5}-Einheit und R₂(CH₂=CH)SiO_{0,5}-Einheit ausgewählt ist, wobei R mit demjenigen aus obigem (1) identisch ist, und durchschnittlich 1,5 oder mehr an ein Siliciumatom gebundene Vinylgruppen im Molekül;
wobei für mindestens eine der Gruppen R in den obigen allgemeinen Gleichungen (1) und (2) 30 Mol-% oder mehr eine Methylgruppe sind und 5-50 Mol-% eine Kohlenwasserstoffgruppe mit 10-22 Kohlenstoffatomen sind;
wobei die durch (1) und (2) dargestellten Organopolysiloxane so gewählt sind, dass der Gesamtgehalt der siliciumatomgebundenen Wasserstoffatome und/oder der siliciumatomgebundenen Vinylgruppen weniger als 20 Mol-% der Summe aller siliciumatomgebundenen organischen Gruppen und Wasserstoffatome beträgt.

2. Zusammensetzung nach Anspruch 1, wobei es sich bei dem Öl, das von einem Silikonöl verschieden ist, um ein Kohlenwasserstofföl handelt.

3. Zusammensetzung nach Anspruch 2, wobei es sich bei dem Kohlenwasserstofföl um ein verzweigtes Kohlenwasserstofföl handelt.

4. Zusammensetzung nach Anspruch 2 oder 3, wobei es sich bei dem Kohlenwasserstofföl um ein flüchtiges Kohlenwasserstofföl handelt.

5. Zusammensetzung nach einem der Ansprüche 1-4, wobei es sich bei dem Öl, das von einem Silikonöl verschieden ist, um ein Esteröl handelt.

6. Zusammensetzung nach einem der Ansprüche 1-4, wobei es sich bei dem Öl, das von einem Silikonöl verschieden ist, um ein Glyceridöl handelt.

7. Zusammensetzung nach einem der Ansprüche 1-4, wobei es sich bei dem Öl, das von einem Silikonöl verschieden ist, um ein natürliches Tier- oder Pflanzenöl und halbsynthetisches Öl handelt.

8. Kosmetische Zubereitung, umfassend die Zusammensetzung nach einem der Ansprüche 1-7 als Komponente A.

9. Kosmetische Zubereitung nach Anspruch 8, enthaltend ein Öl, das von dem in Komponente A) enthaltenen Öl verschieden ist, als Komponente B).

10. Kosmetische Zubereitung nach Anspruch 9, wobei mindestens ein Teil des Öls, das Komponente B) darstellt, bei gewöhnlicher Temperatur flüssig ist.

11. Kosmetische Zubereitung nach Anspruch 9 oder 10, wobei es sich bei mindestens einem Teil des Öls, das Komponente B) darstellt, um ein geradkettiges, verzweigtes oder cyclisches Silikonöl, das durch R₁ₐSiO_{(4-a)/2}, worin R₁ für ein Wasserstofatom oder eine Alkylgruppe mit 1-30 Kohlenstoffatomen, eine Arylgruppe, eine Aralkylgruppe oder eine fluorsubstituierte Alkylgruppe steht und a für eine positive Zahl im Bereich von 1,5≤a≤2,5 steht, dargestellt wird, handelt.

12. Kosmetische Zubereitung nach einem der Ansprüche 9-11, wobei es sich bei mindestens einem Teil des Öls, das Komponente B) darstellt, um ein Öl mit einer Fluorgruppe oder einer Aminogruppe handelt.

13. Kosmetische Zubereitung nach einem der Ansprüche 8-12, enthaltend Wasser als Komponente C).

14. Kosmetische Zubereitung nach einem der Ansprüche 8 bis 13, enthaltend eine Verbindung mit einer alkoholischen Hydroxylgruppe in ihrer Molekülstruktur als Komponente D).

15. Kosmetische Zubereitung nach Anspruch 14, wobei es sich bei der Verbindung mit einer alkoholischen Hydroxylgruppe in ihrer Molekülstruktur als Komponente D) um einen wasserlöslichen einwertigen Alkohol und/oder wasserlöslichen mehrwertigen Alkohol handelt.

16. Kosmetische Zubereitung nach einem der Ansprüche 8-15, enthaltend ein wasserlösliches oder wasserquellendes Polymer als Komponente E).

17. Kosmetische Zubereitung nach einem der Ansprüche 8-16, enthaltend ein Pulver und/oder Farbmittel als Komponente F).

18. Kosmetische Zubereitung nach Anspruch 17, wobei es sich bei mindestens einem Teil des Pulvers und/oder Farbmittels, das Komponente F) darstellt, um ein Feinpulver aus Silikon vom Vernetzungstyp mit einer Struktur, in der Dimethylsilikon vernetzt ist, Polymethylsilsesquioxan-Feinpulver, hydrophobes Siliciumdioxid oder ein Verbundfeinpulver, wobei eine sphärische Silikonkautschukoberfläche mit Polymethylsilsesquioxan-Partikeln überzogen ist, handelt.

19. Kosmetische Zubereitung nach Anspruch 17 oder 18, wobei es sich bei mindestens einem Teil des Pulvers und/oder Farbmittels, das Komponente F) darstellt, um ein Pulver und/oder Farbmittel mit einer Fluorgruppe handelt.

20. Kosmetische Zubereitung nach einem der Ansprüche 8-19, enthaltend ein Tensid als Komponente G).

21. Kosmetische Zubereitung nach Anspruch 20, wobei es sich bei dem Tensid, das Komponente G) darstellt, um ein geradkettiges oder verzweigtes Silikon mit einer Polyoxyalkylenkette im Molekül handelt.

22. Kosmetische Zubereitung nach Anspruch 20 oder 21, wobei der HLB-Wert des Tensids, das Komponente G) darstellt, 2-8 beträgt.

23. Kosmetische Zubereitung nach einem der Ansprüche 8-22, enthaltend ein Organopolysiloxan vom Vernetzungstyp, das mit mindestens seinem Eigengewicht eines niederviskosen Silikonöls mit einer Viskosität von 0,65 mm²/s (25°C) - 100,0 mm²/s aufquillt, als Komponente H).

24. Kosmetische Zubereitung nach Anspruch 23, wobei es sich bei dem Organopolysiloxan vom Vernetzungstyp, das Komponente H) darstellt, um ein Organopolysiloxan vom Vernetzungstyp, das mindestens einen unter einem Polyoxyalkylenteil, Alkylteil, Alkenylteil, Aryteil und Fluoralkylteil ausgewählten Teil in dem vernetzten Molekül enthält, handelt.

25. Kosmetische Zubereitung nach einem der Ansprüche 8-24, enthaltend ein Silikonharz als Komponente I) .

26. Kosmetische Zubereitung nach Anspruch 25, wobei es sich bei dem Silikonharz, das Komponente I) darstellt, um ein Acrylsilikonharz handelt.

27. Kosmetische Zubereitung nach Anspruch 26, wobei es sich bei dem Silikonharz, das Komponente I) darstellt, um ein Acrylsilikonharz, das mindestens einen unter einem Pyrrolidonteil, langkettigen Alkylteil, Polyoxyalkylenteil, Fluoralkylteil und anionischen Teil wie einer Carbonsäure ausgewählten Teil im Molekül enthält, handelt.

28. Kosmetische Zubereitung nach Anspruch 25, wobei es sich bei dem Silikonharz, das Komponente I) darstellt, um eine retikuläre Silikonverbindung, die durch MQ, MDQ, MT, MDT oder MDTQ als Aufbaukomponente dargestellt wird, handelt.

29. Kosmetische Zubereitung nach Anspruch 25 oder 28, wobei es sich bei dem Silikonharz, das Komponente I) darstellt, um eine retikuläre Silikonverbindung, die mindestens einen unter einem Pyrrolidonteil, langkettigen Alkylteil, Polyoxyalkylenteil, Fluoralkylteil und Aminoteil ausgewählten Teil im Molekül enthält, handelt.

## Revendications

1. Composition pâteuse comprenant un organopolysiloxane de type à réticulation qui se dilate par incorporation d'au moins son propre poids d'une huile autre qu'une huile de silicone, et une huile autre qu'une huile de silicone,
**caractérisée en ce que** ledit organopolysiloxane de type à réticulation est un polymère d'organopolysiloxane de type à réticulation obtenu par une polymérisation par addition de l'organohydrogénopolysiloxane exprimé par (1) ci-dessous avec l'organopolysiloxane contenant un groupe vinyle exprimé par (2) ci-dessous,
(1) : un organohydrogénopolysiloxane comprenant au moins un type de motif structurel choisi parmi les groupes comprenant un motif SiO₂, un motif HSiO_{1,5}, un motif RSiO_{1,5}, un motif RHSiO, un motif R₂SiO, un motif R₃SiO_{0,5} et un motif R₂HSiO_{0,5}, dans lequel R est un groupe hydrocarboné monofonctionnel comportant 1 à 30 atomes de carbone qui peut être substitué ou non substitué, mais R ne peut pas être un groupe aliphatique insaturé, et comprenant en moyenne 1,5 atome d'hydrogène ou plus liés à un atome de silicium dans la molécule,
(2) : un organopolysiloxane comprenant au moins un type de motif structurel choisi parmi les motifs suivants : un motif SiO₂ un motif (CH₂=CH)SiO_{1,5}, un motif RSiO_{1,5}, un motif R(CH₂=CH)SiO, un motif R₂SiO, un motif R₃SiO_{0,5} et un motif R₂(CH₂=CH)SiO_{0,5}, dans lequel R est identique à celui du (1) mentionné précédemment, et comprenant en moyenne 1,5 groupe vinyle ou plus liés à un atome de silicium dans la molécule,
dans laquelle, pour l'un au moins des R dans les équations générales (1) et (2) mentionnées précédemment, 30 % en moles ou plus sont un groupe méthyle et 5 à 50 % en moles sont un groupe hydrocarboné comportant 10 à 22 atomes de carbone,
dans laquelle les organopolysiloxanes exprimés par (1) et (2) sont choisis de sorte que la teneur totale de l'un au moins des atomes d'hydrogène liés à un atome de silicium et des groupes vinyle liés à un atome de silicium soit inférieure à 20 % en moles de la somme de tous les groupes organiques et de tous les atomes d'hydrogène liés à un atome de silicium.

2. Composition selon la revendication 1, dans laquelle ladite huile autre qu'une huile de silicone est une huile hydrocarbonée.

3. Composition selon la revendication 2, dans laquelle ladite huile hydrocarbonée est une huile hydrocarbonée ramifiée.

4. Composition selon la revendication 2 ou 3, dans laquelle ladite huile hydrocarbonée est une huile hydrocarbonée volatile.

5. Composition selon l'une quelconque des revendications 1 à 4, dans laquelle ladite huile autre qu'une huile de silicone est une huile d'ester.

6. Composition selon l'une quelconque des revendications 1 à 4, dans laquelle ladite huile autre qu'une huile de silicone est une huile de glycéride.

7. Composition selon l'une quelconque des revendications 1 à 4, dans laquelle ladite huile autre qu'une huile de silicone est une huile naturelle animale ou végétale et une huile semi-synthétique.

8. Préparation cosmétique comprenant la composition selon l'une quelconque des revendications 1 à 7 en tant que composant A).

9. Préparation cosmétique selon la revendication 8, contenant une huile autre que l'huile qui est contenue dans le composant A), en tant que composant B).

10. Préparation cosmétique selon la revendication 9, dans laquelle au moins une partie de l'huile qui est le composant B) est liquide à température ordinaire.

11. Préparation cosmétique selon la revendication 9 ou 10, dans laquelle au moins une partie de l'huile qui est le composant B) est une huile de silicone à chaîne droite, ramifiée ou cyclique, exprimée par R₁ₐSiO_{(4-a)/2}, dans laquelle R₁ est un atome d'hydrogène ou un groupe alkyle comportant 1 à 30 atomes de carbone, un groupe aryle, un groupe aralkyle ou un groupe alkyle substitué par un atome de fluor, et a est un nombre entier positif compris dans la plage de 1,5 ≤ a ≤ 2,5.

12. Préparation cosmétique selon l'un quelconque des revendications 9 à 11, dans laquelle au moins une partie de l'huile qui est le composant B) est une huile contenant un atome de fluor ou un groupe amino.

13. Préparation cosmétique selon l'un quelconque des revendications 8 à 12, contenant de l'eau en tant que composant C).

14. Préparation cosmétique selon l'un quelconque des revendications 8 à 13, contenant un composé comprenant un groupe hydroxyle alcoolique dans sa structure moléculaire en tant que composant D).

15. Préparation cosmétique selon la revendication 14, dans laquelle le composé comprenant un groupe hydroxyle alcoolique dans sa structure moléculaire en tant que composant D) est un alcool monohydrique soluble dans l'eau et/ou un polyol soluble dans l'eau.

16. Préparation cosmétique selon l'un quelconque des revendications 8 à 15, contenant un polymère soluble dans l'eau ou se dilatant dans l'eau en tant que composant E).

17. Préparation cosmétique selon l'un quelconque des revendications 8 à 16, contenant une poudre et/ou un colorant en tant que composant F).

18. Préparation cosmétique selon la revendication 17, dans laquelle au moins une partie de ladite poudre et/ou dudit colorant qui est le composant F) est une poudre fine de silicone de type à réticulation ayant une structure dans laquelle de la diméthylsilicone est réticulée, une poudre fine de polyméthylsilsesquioxane, de la silice hydrophobe ou une poudre fine composite dans laquelle une surface de caoutchouc de silicone sphérique est revêtue de particules de polyméthylsilsesquioxane.

19. Préparation cosmétique selon la revendication 17 ou 18, dans laquelle au moins une partie de la poudre et/ou du colorant qui est le composant F) est une poudre et/ou un colorant comprenant un groupe fluor.

20. Préparation cosmétique selon l'un quelconque des revendications 8 à 19, contenant un tensioactif en tant que composant G).

21. Préparation cosmétique selon la revendication 20, dans laquelle le tensioactif qui est le composant G) est une silicone à chaîne droite ou ramifiée comprenant une chaîne polyoxyalkylène dans la molécule.

22. Préparation cosmétique selon la revendication 20 ou 21, dans laquelle le HLB du tensioactif qui est le composant G) est de 2 à 8.

23. Préparation cosmétique selon l'un quelconque des revendications 8 à 22, contenant un organopolysiloxane de type à réticulation qui se dilate avec au moins son propre poids d'une huile de silicone de faible viscosité ayant une viscosité de 0,65 mm²/s (25°C) à 100,0 mm²/s (25°C) en tant que composant H).

24. Préparation cosmétique selon la revendication 23, dans laquelle l'organopolysiloxane de type à réticulation qui est le composant H) est un organopolysiloxane de type à réticulation contenant au moins une partie choisie parmi les parties suivantes : une partie polyoxyalkylène, une partie alkyle, une partie alcényle, une partie aryle et une partie fluoroalkyle dans la molécule réticulée.

25. Préparation cosmétique selon l'un quelconque des revendications 8 à 24, contenant une résine de silicone en tant que composant I).

26. Préparation cosmétique selon la revendication 25, dans laquelle la résine de silicone qui est le composant I) est une résine de silicone acrylique.

27. Préparation cosmétique selon la revendication 26, dans laquelle la résine de silicone qui est le composant I) est une résine de silicone acrylique contenant au moins une partie choisie parmi les parties suivantes : une partie pyrrolidone, une partie alkyle à chaîne longue, une partie polyoxyalkylène, une partie fluoroalkyle et une partie anionique telle qu'un acide carboxylique dans la molécule.

28. Préparation cosmétique selon la revendication 25, dans laquelle la résine de silicone qui est le composant I) est un composé de silicone réticulaire exprimé par MQ, MDQ, MT, MDT ou MDTQ en tant que composant structurel.

29. Préparation cosmétique selon la revendication 25 ou 28, dans laquelle la résine de silicone qui est le composant I) est un composé de silicone réticulaire contenant au moins une partie choisie parmi les parties suivantes : une partie pyrrolidone, une partie alkyle à chaîne longue, une partie polyoxyalkylène, une partie fluoroalkyle et une partie amino dans la molécule.
